# EUROPEAN PATENT APPLICATION

(11) **EP 0 987 020 A1**
(43) Date of publication of application: **22.03.2000**
(21) Application number: 98402192.3
(22) Date of filing: 04.09.1998
(51) Int. Cl.: A61K 9/20

(54) **Metoprolol composition and processes for manufacturing the same**

(71) Applicant: Pharma Pass LLC, Irvine, CA 92718 (US)
(72) Inventor: Seth, Pawan, Irvine, California, CA 92612 (US)
(74) Representative: Pochart, François

(57) **Abstract**

The present invention relates to a solid metoprolol composition, containing polyethylene oxide and metoprolol, and to methods for their preparation.

## Description

The present invention relates to a novel solid metoprolol composition, containing polyethylene oxide and metoprolol, and to methods for their preparation.

Certain medicaments need to be formulated in so-called delayed-release or sustained release form.

Polyethylene oxide referred to as PEO below is moreover known as a component of medicaments in tablet form designed to be administered by oral route. This compound is marketed by the Union Carbide Corporation under the commercial name Polyox. The use of PEO for formulating medicaments has furthermore been the subject matter of many earlier patents.

EP-A-0 277 092, in the name of Ciba-Geigy relates to a composition comprising a casing in a material that is porous to water but not to the active ingredient, surrounding a central core consisting of a mixture of a substance that is weakly soluble in water, and a hydrophilic swelling material, said material consisting of a mixture of PEO and a vinyl pyrrolidone/vinyl acetate polymer. The composition in that patent is an example of current compositions in which a core which swells when exposed to water is surrounded by a water-porous material, release of the active ingredient being delayed or sustained as a result of the time necessary to expand the core, and for diffusion to take place through the casing following the penetration of water.

The abstract of the US-A-4,404,183 and US-A-4,343,789 discloses two embodiments of a sustained release composition. In the first embodiment, the composition contains PEO, the active ingredient in an amorphous form, and a basic component. In the second embodiment, the active ingredient is nicardipine in an amorphous state, it being possible to omit the basic component.

Actually, the compositions according to the prior art are complex, require specific active ingredients or are provided in a specific form. Moreover, the results achieved are not always very good.

The present invention provides a simple composition that is suitable for use with metoprolol, and has a remarkable delaying or sustaining effect.

Thus, the present invention provides a solid composition comprising, by weight based on the total weight of the composition:
(a) from 1 to 70% of metoprolol which is not in an amorphous form;
(b) from 10 to 95% of polyethylene oxide;
(c) the balance consisting of conventional additives, excluding basic components.

The expression "solid composition" means that the composition is in a tablet or mini-tablet form, these in their turn being able to be encapsulated using for example the conventional hard gelatin.

Metoprolol active ingredient is intended to cover especially the salts of metoprolol, such as succinate and tartrate. Metoprolol tartrate is the preferred active ingredient.

The expression "not in amorphous form" should be understood in its conventional meaning. Various sources give a definition of this term "amorphous" as meaning non-crystalline, lacking the lattice structure characterizing the crystalline state. The following references, which provide a definition of the term amorphous (or the opposite thereof) are, in a non-limiting manner: Hawley's, Condensed Chemical Dictionary, 12th Edition, p. 71; Handbook of Chemistry and Physics, 65th Edition, F-67; The Theory and Practice of Industrial Pharmacy, 1970, pp. 253-255; Remington's Pharmaceutical Sciences, 14th Edition, p. 182; General Chemistry 1992, pp.314-315; Encyclopedia of Pharmaceutical Technology, vol I, pp. 12-13.

The expression "excluding basic compounds" should be understood as excluding the presence of a compound or a group of compounds that confer a basic nature on the composition, in other words a pH greater than 7, when the composition is diluted in water at a rate of 10g per liter of water. In particular, this term should be taken as excluding the presence of one or several basic component(s) such as described in column 1, lines 38 to 62 of US-A-4,404,183 if no acid compound is counteracting the effect of said basic compound, or if the basic compound is present in a relatively large amount.

According to one preferred embodiment, the composition according to the invention, comprises:
(a) from 5 to 45% of metoprolol active ingredient;
(b) from 25 to 70% polyethylene oxide
(c) the balance consisting of conventional additives, excluding basic components.

According to one alternative embodiment, in the composition according to the invention, the polyethylene oxide has a molecular weight which varies from 50,000 to 8,000,000, and preferably from 100,000 to 3,000,000. The required molecular weight for the PEO can be obtained by mixing PEO of differing molecular weights, that are available commercially.

According to a further embodiment, in the composition according to the invention, the balance consisting of conventional additives comprises microcrystalline cellulose, lactose, ascorbic acid, pigments, plasticizing agents, lubricants and so on. Obviously, other conventional additives known to those skilled in the art can be employed.

According to one embodiment of the invention, in a composition, the balance consisting of conventional additives comprises magnesium stearate and/or glycerol behenate and/or sodium stearyl fumarate, which is employed as a lubricant ensuring better compression of the composition when provided in tablet form, for example.

According to one or alternative embodiment, the composition is additionally coated. Surface coating is employed for the purposes of improving appearance making the drug more readily acceptable to the patient, or for dimensionally stabilizing the compressed tablet. The coating can be a conventional coating suitable for enteral use. It is obtained using any conventional technique employing conventional ingredients. A surface coating can for example be obtained using a quick-dissolving film. It should be noted that the coating according to this invention is fundamentally different from the coating used in EP-A-0,277,092 as one does not encounter, in the invention, the dichotomy (water-swellable core)/(water-porous coating), and moreover, the coating in the invention dissolves and/or disintegrates whereas the coating in EP-A-0,277,092 does not dissolve.

The present solid compositions are suitable for the administration of medicaments. Thus, the invention also relates to pharmaceutical compositions deriving therefrom as well as to compositions thereof for use as medicaments.

The present composition can be obtained by any conventional method known to those skilled in the art such as, for example direct compression after simply mixing the dry ingredients, moist or wet granulation involving the use of a granulation liquid, and dry granulation involving a densification phase for the dry mixture.

However, use is preferably made of a process comprising the steps of:
(i) mixing in the dry state and for a sufficient time, the metoprolol active ingredient, polyethylene oxide and optionally, one or several additives;
(ii) optionally adding solvent when this is used, followed by mixing for a sufficient period of time;
(iii) granulation;
(iv) drying the granules thus formed for a sufficient period of time;
(v) optionally adding one of more additives, with mixing in the dry state for a sufficient time and passage through a suitable sieve;
(vi) optionally adding one or several additives and mixing in the dry state for a sufficient period of time;
(vii) compressing the mixture obtained from the preceding steps to obtain the desired compressed tablet; and
(viii) optionally coating said compressed tablet.

The solvent employed, when use is made of a solvent, is preferably an alcohol. The solvent is eliminated by drying at one point or another in the process, and is substantially not encountered in the final composition.

Granulation can be performed by passage through a suitable sieve.

The drying step can be implemented e.g. by using a fluidized bed or a drying oven.

The above granulation and drying steps can be combined by using a fluidized bed granulator (e.g. with a top spray chamber).

The choice of mixing times, apparatus used, sieve mesh, and other operating conditions are within the province of the normal knowledge of those skilled in the art.

Without wishing to be bound by any theory, the applicant believes that the PEO, in the formulation, forms a hydrogel from contact with water. This hydrogel dissolves more or less rapidly as a function of the molecular weight of the PEO employed. Choosing the molecular weight of the PEO, in combination with a suitable choice of the weight concentrations of the active ingredient, of PEO, and of additives enables release of the active ingredient to be controlled.

The carrying out of the process as depicted above with water as the wetting liquid is difficult. Indeed, PEO is a water-swellable polymer which instantaneously forms agglomerates when it comes into contact with the droplets of water. This leads to the final formation of elastic lumps within the heart of the mass to be granulated, these being difficult to size, notably on an oscillating granulation grid.

The instant improved process is thus a method for wet granulation of PEO, using water as the wetting liquid. It is now possible to avoid both the problems linked to direct compression (the heterogeneous nature of the mixture) as well as problems associated with organic solvents (cost, inflammability, and toxicity towards the operator and the environment).

The improved process consists in a two-stage wetting allowing PEO to be hydrated without forming lumps. During the first wetting step, aqueous wetting of the active ingredient alone is carried out or, if appropriate, of the active ingredient and one or several excipients such as, for example, microcrystalline cellulose or lactose. During the second wetting step, the PEO is added to the foregoing wet mixture. These two wetting steps are both simply referred to as the "wetting step" herein.

The wetting liquid can if necessary include substances in solution in order to modify the properties of the granulated product, and to improve the wetting operation. Thus, binders such as hydroxypropylmethyl cellulose or polyvinylpyrrolidone can be employed in order to improve the properties of the granulated product. When such substances are used, their concentration can vary up to about 20%, for example between 1 and 10%.

It is important to note that in all cases, water constitutes substantially the only solvent of the wetting liquid in the improved process.

The wetting operation can be carried out in any conventional blender described in the literature such as for example apparatus manufactured by Vector, Moritz, Collette, Bouvard, Lodige, etc. Such blenders can employ kneading, blades, planetary arrangements or, preferably but not obligatorily, high speed with or without a lump-breaking knife. Wetting takes place for a period comprised between 0,5 and 15 min, for example between 2 and 6 min.

Preferably, but not indispensably, slight "over-wetting" of the powders is done which renders the following operations easier. "Over-wetting" should be taken to mean "adding liquid in excess to the amount required to agglomerate the powder mixture".

The excess water added during the possible slight over-wetting in the first step improves agglomeration of the PEO to the active ingredient and, if present, to the other excipients present.

For example, the weight ratios water/composition or water/PEO may vary respectively from 0.13 to 0.36 and 0.18 to 0.5.

Once the first wetting step has been performed, the PEO is added into the apparatus and a supplementary mixing is performed thereby achieving supplementary wetting of the PEO. During this second mixing, the PEO gets integrated into the granulated product and swells slightly. However, surprisingly, there is no formation of lumps or clods, contrary to what is obtained in conventional granulation, using one single step, as described in the literature.

This last operation can be carried out in the same mixer as was used for the first step, or any other conventional blender as described above. The mixing time is comprised between 0,5 and 15 min, for example between 2 and 6 min.

The process includes other steps which are conventional in the art.

One prior step in which the active ingredient is intimately mixed with the additives, when present, can be provided.

A granulation step notably is carried out after incorporation and mixing of the PEO. This granulation step can be done on a screen having a suitable mesh size.

A drying step, notably using a fluidized bed or a drying oven, is also implemented, notably following the granulation step. The drying step is considered as having been carried out when the residual water content is below 10%, for example below 3%, depending on the kind and amount of ingredients .

The above granulation and drying steps can be combined by using a fluidized bed granulator (e.g. with a top spray chamber).

Steps in which additives are added and blended can be included, if necessary.

A step in which the granules, notably dry granules, are size-graded can be provided. Size grading is carried out also on a screen having a mesh size comprised for example between 100 and 1000 µm.

Final compression steps and, optionally, tablet coating steps are also provided. Notably, a step in which a compression additive is added can be provided after drying, but prior to compression. These final compression steps are obviously not necessary for preparing capsules enclosing the granules.

It is clear that the above order for the steps, apart from that of wetting, is not fixed; one could for example add the compression additives right at the beginning or immediately following addition of the PEO. Similarly, a granulation step could optionally be carried out before adding the PEO.

Choice of the duration of mixing, the apparatus, the screens and other operating conditions is a matter for the normal skill and judgement of the person skilled in the art.

The improved process is thus a process for preparing a solid pharmaceutical composition comprising, by weight based on the total weight of the composition:
(a) from 1 to 70% of active ingredient which is not in an amorphous form;
(b) from 10 to 95% of polyethylene oxide;
(c) the balance consisting of conventional additives, comprising the steps of:
   (i) mixing said active ingredient and optionally one or several additives with an aqueous solution for a sufficient period of time; then
   (ii) adding the PEO, and mixing for a sufficient period of time.

According to one embodiment, step (i) of mixing with the aqueous solution is carried out in the presence of excess liquid.

According to another embodiment, said aqueous solution consists of water, and, optionally, water-soluble additives.

According to yet another embodiment, the improved process further comprises a granulation step; said granulation step can be carried out on a screen of suitable mesh size or using a fluidized bed granulator.

According to yet another embodiment, the improved process additionally comprises a drying step.

According to yet another embodiment, the improved process additionally comprises a compression step; said compression step can be preceded by a step in which additives are added and blended.

A specific embodiment of the instant improved process is a process comprising the steps of:
(i) mixing, for a sufficient period of time, active ingredient and, optionally, one of several additives, with said aqueous solution; then
(ii) adding PEO and mixing for a sufficient period of time;
(iii) granulating the mixture obtained from step (ii) ;
(iv) drying granules thus formed for a sufficient period of time;
(v) optionally, adding one or several additives, and mixing in the dry state for a sufficient period of time;
(vi) compressing the mixture obtained from the preceding step into a tablet.

Preferably, step (i) of mixing with the aqueous solution is carried out in the presence of excess liquid.

Preferably steps (iii) and (iv) are carried out as a single step in a fluidized bed granulator.

Another specific embodiment of the invention corresponds to the improved process where granulation (and drying) steps are carried out before the PEO is added. Mixing, granulating (and drying) are preferably carried out at once, e.g. in a fluidized bed granulator.

Said alternate specific embodiment of the instant improved process is a process comprising the steps of:
(i) mixing, for a sufficient period of time, active ingredient and, optionally, one of several additives, with said aqueous solution; then
(ii) granulating the mixture obtained from step (i) ;
(iii) optionally drying the granules thus formed for a sufficient period of time;
(iv) adding PEO and optionally one or several additives, and mixing for a sufficient period of time;
(v) compressing the mixture obtained from the preceding step into a tablet.

Preferably, steps (i) to (iii) are carried out as a single step in a fluidized bed granulator.

Preferably, the aqueous solution comprises water and a binder in an amount up to 20%.

Said alternate embodiment can be applied not only to metoprolol, but to any other active ingredient.

The examples below are provided as examples illustrating the invention and should not be considered as limiting its scope. In the examples, although the amount water employed is given, it should be understood that the water is substantially absent in the final composition. In the examples, tablet hardness is measured using a Schleuniger 4M hardness tester.

### Example 1:

The following formulation was prepared

| uncoated tablet (in mg) | |
|---|---|
| Metoprolol salt | 200 |
| Polyox WSR N 301 (MW=4000000) | 300 |
| Avicel PH 101 | 54 |
| Plasdone K29-32 | 8 |
| Purified water | 60 |
| Aerosil 200 | 3 |
| Sodium stearyl fumarate | 5 |

The manner of preparation was as follows. Metoprolol and Avicel were introduced into a Stephan UMC5 blender, and blending was carried out for 5 minutes. The Plasdone K29-32 was dissolved in water, and the powder was wetted with this solution. Blending was continued for 5 minutes. Next, the Polyox was added and blending was continued for a further 5 minutes. Granulation was performed on an oscillating Erweka FGS granulator provided with a 1.6 mm mesh grid. The product was dried on a fluidized bed down to a constant humidity. Size-grading was carried out on an oscillating granulator with a 0.8 mm mesh grid. The Aerosil 200, any lumps previously being broken up, and the sodium stearyl fumarate were added. Blending was continued for 5 minutes. Next, the powder was compressed on a rotary Fette P2 press provided with 12 mm diameter and 12 mm radius of curvature punches. The nominal mass of the tablet was 570 mg and its average hardness was set to 18 kg. These tablets were then given a film coating using a conventional formulation (e.g. Opadry).

### Example 2:

The following formulation was prepared

| uncoated tablet (in mg) | |
|---|---|
| Metoprolol tartrate | 200 |
| Polyox coagulant | 303 |
| Avicel PH 302 | 100 |
| Plasdone K29-32 | 16 |
| Aerosil 200 | 2 |
| Sodium stearyl fumarate | 9 |
| Purified water | 200 |

The manner of preparation was as follows. Metoprolol tartrate, Avicel and Aerosil were introduced into a Stephan UMC5 blender, and blending was carried out for 5 minutes. The Plasdone K29-32 was dissolved in water. The powder mixture was granulated with the solution in a fluidized bed granulator (with a top spray chamber), using the following spraying parameters:

| | |
|---|---|
| Air flow (m³/h) | 65 m³/h |
| Liquid flow (g/min) | 16 g/min |
| Inlet temperature | 65°C |
| Spraying pressure | 2.0 bar |

Next, the Polyox and the sodium stearyl fumarate were added and blending was continued for a further 5 minutes. Next, the powder was compressed on a rotary Fette P2 press provided with 12 mm diameter and 12 mm radius of curvature punches. These tablets were then given a film coating using a conventional formulation (e.g. Opadry).

## Claims

1. Solid composition comprising, by weight based on the total weight of the composition:
(a) from 1 to 70% of metoprolol active ingredient which is not in an amorphous form;
(b) from 10 to 95% of polyethylene oxide;
(c) the balance consisting of conventional additives, excluding basic components.

2. The composition of claim 1, wherein the metoprolol active ingredient is metoprolol tartrate.

3. The composition of claim 1 or 2, comprising:
(a) from 5 to 45% of metoprolol active ingredient;
(b) from 25 to 70% polyethylene oxide
(c) the balance consisting of conventional additives, excluding basic components.

4. The composition according to any one of claims 1 to 3, in which the polyethylene oxide has a molecular weight which varies from 50,000 to 8,000,000, and preferably from 100,000 to 3,000,000.

5. The composition according to any one of claims 1 to 4, in which the balance consisting of conventional additives comprises microcrystalline cellulose, lactose, ascorbic acid, pigments, plasticizing agents, lubricants and so on.

6. The composition according to any one of claims 1 to 5, which is additionally coated.

7. Process for preparing the composition according to any one of claims 1 to 6, comprising the steps of
(i) mixing in the dry state and for a sufficient time, the metoprolol active ingredient, polyethylene oxide and optionally, one or several additives;
(ii) optionally adding solvent when this is used, followed by mixing for a sufficient period of time;
(iii) granulation;
(iv) drying the granules thus formed for a sufficient period of time;
(v) optionally adding one of more additives, with mixing in the dry state for a sufficient time and passage through a suitable sieve;
(vi) optionally adding one or several additives and mixing in the dry state for a sufficient period of time;
(vii) compressing the mixture obtained from the preceding steps to obtain the desired compressed tablet; and
(viii) optionally coating said compressed tablet.

8. The process according to claim 7, in which the granulation is carried out by passage through a suitable sieve.

9. The process according to claim 7 or 8, in which the solvent employed, when solvent is used, is an alcohol.

10. Process for preparing the composition according to any one of claims 1 to 6, comprising the steps of:
(i) mixing said metoprolol active ingredient and optionally one or several additives with an aqueous solution for a sufficient period of time; then
(ii) adding the PEO, and mixing for a sufficient period of time.

11. The process according to claim 10, in which step (i) of mixing with the aqueous solution is carried out in the presence of excess liquid.

12. The process according to claim 10 or 11, further comprising a granulation step.

13. The process according to claim 12, in which said granulation step is carried out on a screen of suitable mesh size.

14. The process according to claim 12, in which said granulation step is carried out using a fluidized bed granulator.

15. The process according to any one of claims 10 to 14, additionally comprising a drying step.

16. The process according to any one of claims 12, 14 or 15, in which the granulation and drying steps are combined by using a fluidized bed granulator.

17. The process according to any one of claims 10 to 16, further comprising a compression step.

18. The process according to claim 17, in which said compression step is preceded by a step in which additives are added and blended.

19. Process for preparing the composition according to any one of claims 1 to 6, comprising the steps of:
(i) mixing, for a sufficient period of time, metoprolol active ingredient and, optionally, one of several additives, with an aqueous solution; then
(ii) adding PEO and mixing for a sufficient period of time;
(iii) granulating the mixture obtained from step (ii) ;
(iv) drying granules thus formed for a sufficient period of time;
(v) optionally, adding one or several additives, and mixing in the dry state for a sufficient period of time;
(vi) compressing the mixture obtained from the preceding step into a tablet.

20. The process according to claim 19, in which step (i) of mixing with the aqueous solution is carried out in the presence of excess liquid.

21. The process according to claim 19 or 20, in which the granulation and drying steps are combined by using a fluidized bed granulator.

22. Process for preparing the composition according to any one of claims 1 to 6, comprising the steps of:
(i) mixing, for a sufficient period of time, active ingredient and, optionally, one of several additives, with said aqueous solution; then
(ii) granulating the mixture obtained from step (i) ;
(iii) optionally drying the granules thus formed for a sufficient period of time;
(iv) adding PEO and optionally one or several additives, and mixing for a sufficient period of time;
(v) compressing the mixture obtained from the preceding step into a tablet.

23. The process according to claim 22, in which the mixing, granulation and drying steps are combined by using a fluidized bed granulator.

24. The process according to any one of claims 10 to 23, in which said aqueous solution consists of water, and, optionally, water-soluble additives.

25. The process according to claim 24, in which said aqueous solution comprises water and a binder in an amount up to 20%.
